# EUROPÄISCHE PATENTSCHRIFT

(11) **EP 1 776 976 B1**
(45) Veröffentlichungstag und Bekanntmachung des Hinweises auf die Patenterteilung: **14.12.2011**
(21) Anmeldenummer: 05022871.7
(22) Anmeldetag: 20.10.2005
(51) Int. Cl.: A61M 11/02, A61M 15/00

(54) **Inhalationsgerätekompressor für eine Inhalationstherapievorrichtung**
Compressor for an inhalation device for an inhalation therapy
Compresseur pour un dispositif d'inhalation pour une therapie d'inhalation

(43) Veröffentlichungstag der Anmeldung: 25.04.2007
(73) Patentinhaber: PARI GmbH Spezialisten für effektive Inhalation, 82319 Starnberg (DE)
(72) Erfinder: Luber, Martin, 81379 München (DE); Boehm, Andreas, 86934 Reichling (DE); Kummer, Frank, 84028 Landshut (DE)
(74) Vertreter: HOFFMANN EITLE

(56) Entgegenhaltungen:
- EP-A- 0 712 636
- WO-A-03/077978
- WO-A2-2005/035037
- DE-A1-102004 002 031
- DE-U1-202004 016 317
- US-A1- 2005 103 339
- US-B1- 6 474 960

## Beschreibung

Die vorliegende Erfindung betrifft einen Inhalationsgerätekompressor für eine Inhalationstherapievorrichtung, insbesondere für eine Inhalationstherapievorrichtung vom druckluftbetriebenen Düsentyp.

Im Stand der Technik, zum Beispiel aus EP 0 712 636, sind Düsenvernebler bekannt, die die für die Vernebelung notwendige Druckluft mit Hilfe eines durch einen Elektromotor angetriebenen Kompressors erzeugen. Der Kompressor wird in der Regel an Netzspannung betrieben; jedoch verwenden tragbare Düsenvernebler batteriebetriebene Kompressoren, die leichter sind und deshalb vom Benutzer, auch während einer Inhalation, mitgeführt werden können. Das Mitführen des Kompressors hat häufig einen Körperkontakt zur Folge, bei dem sich Vibrationen des Kompressors unangenehm bemerkbar machen. Die Vibrationen des Kompressors können auch zu einer unerwünschten Geräuschentwicklung führen. Ferner kann ein verhältnismäßig leichter Kompressor bei einem Betrieb durch die Eigenvibration auf Ablageflächen, beispielsweise einer Tischplatte, in Bewegung geraten. Daher ist es grundsätzlich notwendig, die durch den Betrieb des Kompressors hervorgerufenen Vibrationen zu minimieren.

Andererseits ist bei tragbaren Kompressoren das Risiko erhöht, dass der Kompressor bei einem Transport beschädigt wird, beispielsweise durch einen Sturz auf den Boden. Daher ist es ebenso notwendig, den Kompressor und dessen Komponenten vor harten Schlägen und Stößen zu schützen.

Im Bereich der Sauerstofftherapiegeräte ist aus WO2005/035037A2 bekannt, das Gehäuse eines auf einem Transportwagen bewegbaren Geräts so auszugestalten, dass ein Kompressor auf einer oberen Oberfläche einer Kompressorhalterung ruht, die sich von einer Basis des Gehäuses nach oben erstreckt. Dabei ragt ein Antriebsmotor des Kompressors in eine Öffnung der Kompressorhalterung hinein. Zur Halterung des Kompressors sind aber Kompressorhaltereinrichtungen vorgesehen, die zwischen dem Kompressor und dem Gehäuse angeordnet sind. Diese Halteeinrichtungen sind als Spannfedern beschrieben, durch die der Kompressor in seiner Position gehaltert wird.

Vor diesem Hintergrund besteht eine Aufgabe der vorliegenden Erfindung darin, einen Inhalationsgerätekompressor für eine Inhalationstherapievorrichtung bereitzustellen, der dafür ausgelegt ist, sowohl leichte Vibrationsschwingungen eines durch einen Elektromotor angetriebenen Kompressors auf ein erforderliches Maß zu dämpfen als auch gleichzeitig den durch einen Elektromotor angetriebenen Kompressor vor einer Beschädigung durch harte Stöße und Schläge zu schützen.

Die Aufgabe der vorliegenden Erfindung wird gelöst durch einen Inhalationsgerätekompressor mit einer mittels Elektromotor angetriebenen Kompressionseinrichtung und einem Gehäuse mit einer Aufnahmeeinrichtung für die Kompressionseinrichtung und einem Halterungselement zum Haltern der Kompressionseinrichtung in der Aufnahmeeinrichtung, das an der Oberfläche der Kompressionseinrichtung angreift und das eine Mehrzahl von Dämpfungsvorsprüngen aufweist, die jeweils eine derart nicht-lineare Kraft-Weg-Kennlinie aufweisen, dass das Kraft/Weg-Verhältnis bei einer Auslenkung der Kompressionseinrichtung zunimmt, wobei das Halterungselement die Kompressionseinrichtung entlang wenigstens eines Teilumfangs angreifend umschließt.

Der erfindungsgemäße Inhalationsgerätekompressor erlaubt durch ein Halterungselement zum Haltern der Kompressionseinrichtung in der Aufnahmeeinrichtung einen sicheren Sitz der Kompressionseinrichtung in einem Gehäuse, wobei durch die Mehrzahl von Dämpfungsvorsprüngen mit einer nicht-linearen Kraft-Weg-Kennlinie erreicht wird, dass bei einer verhältnismäßig geringen Auslenkung, beispielsweise einer Vibration, die Bewegungsenergie in den Dämpfungsvorsprüngen aufgefangen wird, ohne an die Aufnahmeeinrichtung bzw. das Gehäuse weitergegeben zu werden. Auf der anderen Seite wird durch die nicht-lineare Kraft-Weg-Kennlinie erreicht, dass bei härteren Stößen, die beispielsweise bei einem Herunterfallen des Inhalationsgerätekompressors auftreten, relativ gesehen eine geringere Auslenkung der Dämpfungsvorsprünge auftritt. So ist bei einem harten Stoß die negative Beschleunigung, die die Kompressionseinrichtung erfährt, wesentlich geringer als die negative Beschleunigung, die das Gehäuse erfährt. Auf diese Weise ist der erfindungsgemäße Inhalationsgerätekompressor sowohl in der Lage betriebsbedingte Vibrationen für einen komfortableren Betrieb abzudämpfen als auch harte Stöße in Folge beispielsweise eines Sturzes aufzufangen und somit eine Kompressionseinrichtung vor einer Beschädigung zu schützen. Das die Kompressionseinrichtung entlang wenigstens eines Teilumfanges umschließend angreifende Halterungselement erlaubt neben den Dämpfungseigenschaften für Vibrationen und harte Stöße auch eine Positionierung der Kompressionseinrichtung, da die teilweise Umschließung eine tangentiale Verschiebung unterbindet.

Gemäß einer vorteilhaften Ausführungsform weisen die Dämpfungsvorsprünge Erstreckungsachsen auf, die im wesentlichen senkrecht zur Oberfläche der Kompressionseinrichtung ausgerichtet sind. Bei einem Stoß oder einem Sturz erfolgt die Kraftwirkung in der Regel in Richtung des Schwerpunktes, der sich bei einer Kompressionseinrichtung in ihrem Inneren befindet. Die Kraftwirkung bei einem Stoß erfolgt im wesentlichen senkrecht zu wenigstens einem Oberflächenteil der Kompressionseinrichtung. Die Dämpfungsvorsprünge werden somit in Richtung ihrer Erstreckungsachse belastet, so dass ihre Kraft-Weg-Kennlinie entsprechend gut eingestellt werden kann, um die Funktion der Abdämpfung eines harten Stoßes gut erfüllen zu können.

Gemäß einer weiteren vorteilhaften Ausführungsform verjüngen sich die Dämpfungsvorsprünge in einem Bereich entlang der Erstreckungsachse in Richtung der Oberfläche der Kompressionseinrichtung. Bei einer geringen Auslenkung der Kompressionseinrichtung, beispielsweise aufgrund von Vibration, erfolgt die stärkste Verformung an den am stärksten verjüngten Bereichen, die vorteilhafterweise an der Oberfläche der Kompressionseinrichtung anliegen. Durch die Verjüngung der Dämpfungsvorsprünge entlang der Erstreckungsachse in Richtung der Oberfläche wird die nicht-lineare Kraft-Weg-Kennlinie durch eine geometrische Ausformung der Auswölbung erreicht. Vorteilhafterweise kann dadurch das Halterungselement aus einem homogenen Material gefertigt werden, da die nicht-lineare Kraft-Weg-Kennlinie durch die geometrische Ausformung erreicht wird.

Gemäß einer weiteren vorteilhaften Ausführungsform sind die am meisten verjüngten Bereiche der Dämpfungsvorsprünge derart ausgelegt, dass sie an der Kompressionseinrichtung angreifen. Eine derartige Ausformung der Dämpfungsvorsprünge erlaubt eine leichte Montage, da sich die Dämpfungsvorsprünge an ihrem am meisten verjüngten Bereich am leichtesten verformen lassen, wodurch ein einfaches Einführen der Kompressionseinrichtung in oder an das Halterungselement ermöglicht wird.

Gemäß einer weiteren vorteilhaften Ausführungsform sind die Dämpfungsvorsprünge in Richtung der Erstreckungsachse verformbar ausgebildet. Auf diese Weise ist eine Aufnahme der Bewegungsenergie bei einer Bewegung der Kompressionseinrichtung gegenüber dem Gehäuse möglich.

Gemäß einer weiteren vorteilhaften Ausführungsform sind die Dämpfungsvorsprünge im wesentlichen aus elastischen Materialien, vorzugsweise aus einem der Materialien Silikon, EPDM oder TPE gefertigt. Diese Materialien weisen eine hohe Elastizität, eine chemische Widerstandsfähigkeit gegenüber Säuren und Laugen, eine einfache Verarbeitbarkeit und eine gute Alterungsbeständigkeit auf.

Gemäß einer weiteren vorteilhaften Ausführungsform sind die Dämpfungsvorsprünge derart nebeneinander angeordnet, dass sie im wesentlichen in Form einer Welle in Richtung des Umfangs bzw. Teilumfangs der Kompressionseinrichtung ausgestaltet sind. Auf diese Weise lässt sich die nicht-lineare Kraft-Weg-Kennlinie der Dämpfungsvorsprünge mit einer platzsparenden Anordnung der Dämpfungsvorsprünge nebeneinander gut vereinbaren. Bei einer Ausgestaltung in Form einer Welle ohne scharfen Kanten oder Kerben kann zusätzlich ein Verschleißen oder Einreißen des Halterungselementes vermieden werden.

Gemäß einer weiteren vorteilhaften Ausführungsform umfassen die Halterungselemente die Kompressionseinrichtung entlang eines Umfangs im wesentlichen vollständig angreifend. Auf diese Weise kann eine verbesserte Positionierung der Kompressionseinrichtung in der Aufnahmeeinrichtung bewirkt werden, bei der eine Dämpfung in jede Richtung erreicht werden kann und eine gleichmäßige Beanspruchung des Halterungselementes auftritt.

Gemäß einer weiteren vorteilhaften Ausführungsform ist das Halterungselement einstückig mit einer im wesentlichen kreisförmigen Öffnung ausgeführt und weist in der Öffnung nach innen gerichtete Dämpfungsvorsprünge auf. Ein derartiges Halterungselement erlaubt eine Aufnahme der Kompressionseinrichtung derart, dass die Elastizität des Halterungselementes ein Abrutschen von der Kompressionseinrichtung verhindert und ein Verlorengehen des Halterungselementes vermieden werden kann.

### Beschreibung der Figuren

Die nachfolgenden Figuren zeigen vorteilhafte Ausgestaltungen des erfindungsgemäßen Inhalationsgerätekompressors; im Einzelnen zeigt:
- Figur 1: einen Inhalationsgerätekompressor gemäß der vorliegenden Erfindung;
- Figur 2: einen Inhalationsgerätekompressor der vorliegenden Erfindung, bei dem die Kompressionseinrichtung entnommen ist und;
- Figur 3a bis 3c: eine Ausführungsform eines Halterungselementes, das in dem erfindungsgemäßen Inhalationsgerätekompressor verwendet wird.

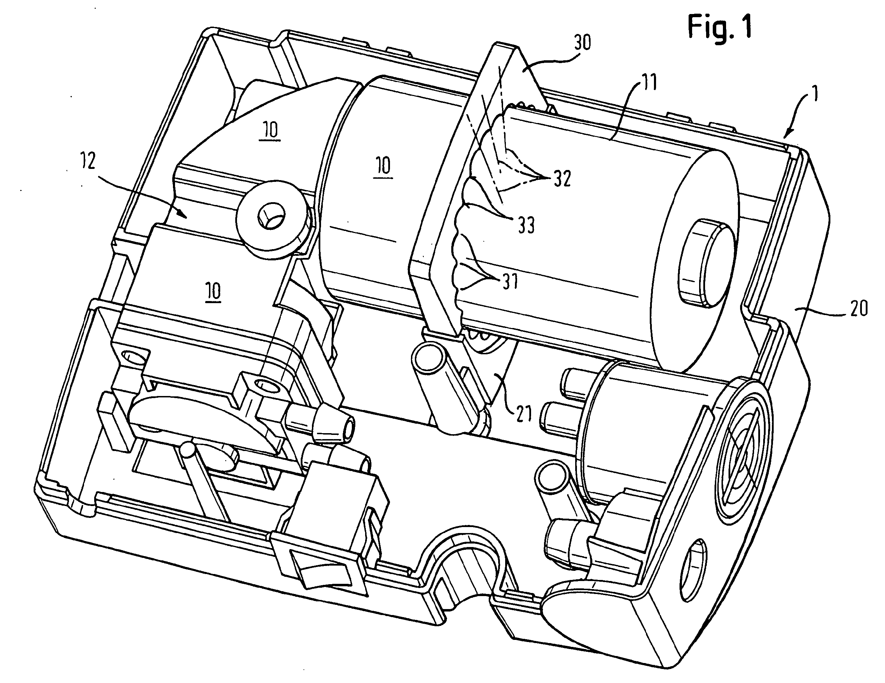

Figur 1 zeigt ein Ausführungsbeispiel eines erfindungsgemäßen Inhalationsgerätekompressors 1. Der Inhalationsgerätekompressor 1 weist ein Gehäuse 20 auf, von dem in Figur 1 nur eine untere Schale gezeigt ist und in dem sich eine mittels Elektromotor 11 angetriebene Kompressionseinrichtung 10 befindet, um für einen hier nicht gezeigten Inhalationsvernebler mit Verneblerdüse Druckluft bereit zu stellen. Das Gehäuse weist eine Aufnahmeeinrichtung 21 auf, um die Kompressionseinrichtung 10 aufzunehmen. Auch von der Aufnahmeeinrichtung 21 ist nur der Teil in Figur 1 gezeigt, der in der dargestellten Gehäuseschale 20 angeordnet ist.

Erfindungsgemäß wird die Kompressionseinrichtung 10 nicht unmittelbar mit der Aufnahmeeinrichtung 21 in Kontakt gebracht, sondern mittels eines Halterungselementes 30 in der Aufnahmeeinrichtung 21 gehaltert. Da eine Gehäuseschale in Figur 1 nicht gezeigt ist, ist das aus der gezeigten Aufnahmeeinrichtung 21 herausragende Teil des Halterungselements 30 gut zu erkennen.

Das Halterungselement 30 greift an der Oberfläche der Kompressionseinrichtung 10 an und weist eine Mehrzahl von Dämpfungsvorsprüngen 31 auf, die jeweils eine nicht-lineare Kraft-Weg-Kennlinie aufweisen, so dass das Kraft/Weg-Verhältnis bei einer Auslenkung der Kompressionseinrichtung 10 aus der Ruhelage zunimmt. Das Halterungselement 30 greift an dem äußeren Umfang des zu der Kompressionseinrichtung 10 gehörenden Elektromotors 11 an. Alternativ kann das Haltungselement auch an einer planen Oberfläche der Kompressionseinrichtung 10 angreifen.

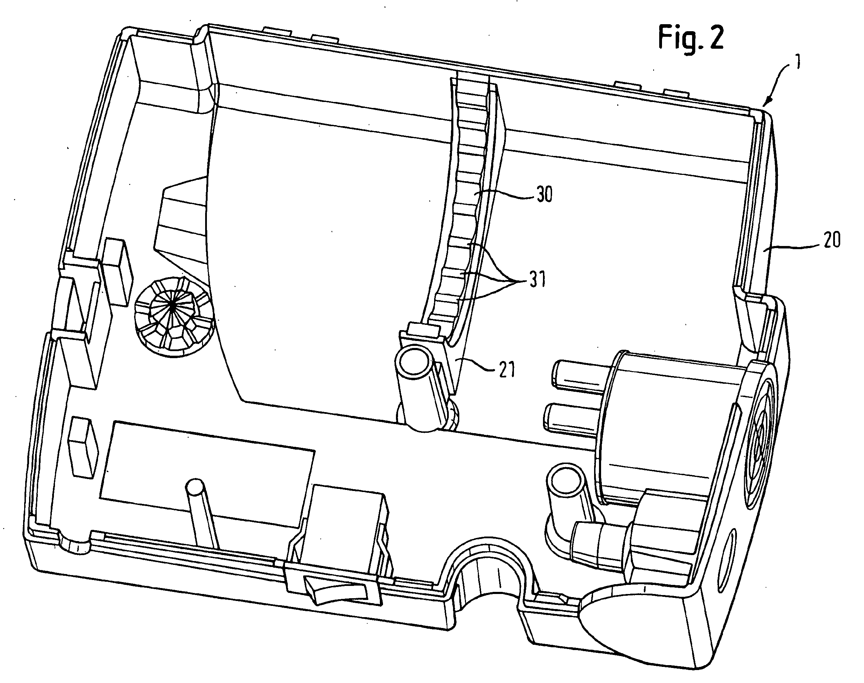

Figur 2 zeigt das Ausführungsbeispiel eines erfindungsgemäßen Inhalationsgerätekompressors gemäß Figur 1, jedoch für eine verbesserte Erkennbarkeit ohne die mittels Elektromotor 11 angetriebene Kompressionseinrichtung 10. Aus der Darstellung der Figur ergibt sich die mögliche Lage und Position der Halterungselemente 30 zum Haltern der Kompressionseinrichtung 10.

In der gezeigten Ausführungsform greift das mit der Bezugsziffer 30 bezeichnete Halterungselement an einem annähernd kreisförmigen Umfang eines zu der Kompressionseinrichtung 10 gehörigen Elektromotors 11 an und umfasst diesen etwa an seinem halben Umfang. Ein entsprechendes Gegenstück einer Aufnahmeeinrichtung 21 und eines Halterungselementes 30 ist in dem in den Figuren 1 und 2 nicht gezeigten Teil des Gehäuses 20 vorgesehen, so dass das in dem Gehäuse 20 vorgesehene Halterungselement und das in dem Deckel des Gehäuses vorgesehene Halterungselement 30 den Elektromotor 11 nahezu vollständig umfassen. Alternativ ist jedoch auch eine Ausführungsform denkbar, bei der nur ein Teilumfang beispielsweise des Elektromotors gehaltert wird, und die Halterungselemente 30 nur an Teilumfängen des zu der Kompressionseinrichtung 10 gehörigen Elektromotors 11 angreifen. Ferner ist ein Halterungselement 30 möglich, das einstückig ausgeführt ist und im Inneren eine im wesentlichen kreisförmige Öffnung aufweist, in die sich die Dämpfungsvorsprünge 31 hinein erstrecken, so dass beispielsweise ein Elektromotor in diese Öffnung zunächst eingeführt wird, um dann zusammen mit dem Halterungselement 30 in dem Gehäuse 20 platziert zu werden.

Die Dämpfungsvorsprünge 31 verjüngen sich entlang ihrer Erstreckungsachsen und greifen in der hier gezeigten Ausführungsform mit den am meisten verjüngten Bereichen 33 an der Kompressionseinrichtung 10 an. Die Dämpfungsvorsprünge 31 sind in Richtung ihrer Erstreckungsachsen 32 verformbar ausgebildet, so dass sie eine durch eine Vibration oder einen Stoß entstehende Bewegungsenergie aufnehmen können und diese beispielsweise in Wärme oder in kinetische Energie umwandeln.

Die nicht-lineare Kraft-Weg-Kennlinie, die zu einem zunehmenden Kraft/Weg- Verhältnis bei einer Auslenkung der Kompressionseinrichtung 10 in Bezug auf das Gehäuse 20 bzw. die Aufnahmeeinrichtung 21 ausführt, kann allein durch die geometrische Ausgestaltung in Form der verjüngenden Dämpfungsvorsprünge 31 herbeigeführt werden. Auf diese Weise ist es möglich, sowohl kleinere Auslenkungen bei einer betriebsbedingten Vibration der Kompressionseinrichtung 10 aufzunehmen als auch die bei einem Stoß oder einem Sturz auftretenden Kräfte aufzunehmen. Die bei einer verhältnismäßig geringen Krafteinwirkung infolge einer Vibration auftretende Auslenkung ist absolut zwar geringer, jedoch relativ gesehen höher als eine Auslenkung, die infolge eines harten Stoßes oder eines Sturzes auftritt. Dies ist im wesentliche darauf zurückzuführen, dass sich bei einer zunehmenden Auslenkung zunächst die am meisten verjüngten Bereiche der Dämpfungsvorsprünge stärker verformen, wobei sich die breiteren Bereiche der Dämpfungsvorsprünge erst bei einer größeren Krafteinwirkung stärker verformen. Auf diese Weise kann durch die geometrische Ausformung der Dämpfungsvorsprünge eine nicht lineare Kraft-Weg-Kennlinie erreicht werden.

Die Dämpfungsvorsprünge bestehen vorteilhafterweise aus elastischen Materialien wie Silikon, Ethylenpropylendimerisaten (EPDM) oder thermoplastischen Elastomeren (TPE). Es sind jedoch darüber hinaus noch andere geeignete Materialien denkbar, die eine elastische Eigenschaft aufweisen.

Je nach Verwendung kann das Halterungselement 30 unterschiedliche Ausgestaltung aufweisen, beispielsweise in Form eines an einem äußeren Umfang beispielsweise eines Zylinders angreifenden Halterungselements, wobei der Zylinder nicht notwendigerweise ein Kreiszylinder sein muss, sondern beispielsweise auch einen ovalen, quadratischen, rechteckigen oder andersförmigen Querschnitt aufweisen kann.

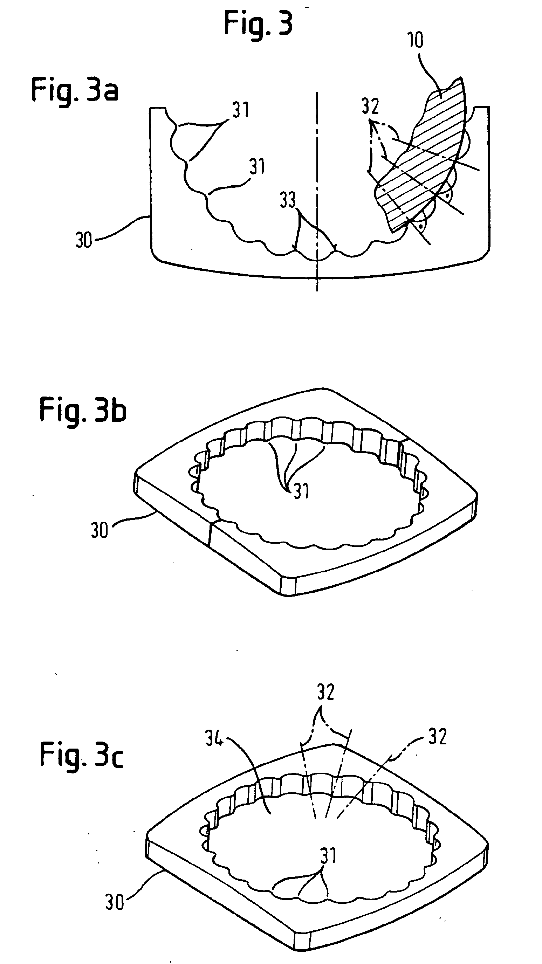

Figur 3 zeigt eine detailliertere Ansicht eines Halterungselementes 30, das derart ausgebildet ist, dass es zumindest an einem Teilumfang der Kompressionseinrichtung 10 angreift.

Figur 3a zeigt eine Schnittansicht eines Halterungselementes, bei dem sich die Dämpfungsvorsprünge 31 entlang einer Erstreckungsachse 32 erstrecken, wobei sich die Erstreckungsachsen 32 im wesentlichen senkrecht zur Oberfläche der Kompressionseinrichtung 10 befindet.

Die Dämpfungsvorsprünge 31 verjüngen sich dabei entlang ihrer Erstreckungsachsen 32 in Richtung der Oberfläche der Kompressionseinrichtung 10. Dabei sind die am meisten verjüngten Bereiche 33 der Dämpfungsvorsprünge 31 so ausgelegt, dass sie an der Kompressionseinrichtung 10 angreifen.

Figur 3b zeigt zwei zusammengefügte Halterungselemente 30, die beispielsweise vorgesehen werden können, wenn eine Kompressionseinrichtung 10 durch zwei jeweils in einem Gehäuseteil vorgesehene Halterungselemente 30 gehaltert werden soll.

Figur 3c zeigt ein Halterungselement, das einstückig mit einer im wesentlichen kreisförmigen Öffnung 34 vorgesehen ist, bei dem die Dämpfungsvorsprünge 31 nach innen gerichtet ausgestaltet sind. Eine derartige Halterungseinrichtung 30 wird vorzugsweise vor einem Einbringen in einem Inhalationsgerätekompressor an der Kompressionsvorrichtung 10 angebracht, um dann gemeinsam mit dieser in der Aufnahme bzw. den Aufnahmen des Gehäuses des Inhalationsgerätekompressors angeordnet zu werden.

Die Dämpfungsvorsprünge 31 sind in dem Halterungselement 30 vorzugsweise nebeneinander angeordnet, so dass ihre der Kompressionseinrichtung zugewandten Oberfläche im wesentlichen in Form einer Welle ausgestaltet sind. Eine derartige Welle kann beispielsweise im wesentlichen die Form einer Sinuswelle aufweisen, jedoch ist auch eine Wellenform denkbar, die sich in Form eines Dreiecks oder Sägezahns bzw. einer Trapezform periodisch wiederholt, so dass der einzelne Dämpfungsvorsprung 31 die Form eines Dreiecks, einer Sinuswelle oder eines Trapezes aufweisen kann, die jeweils aufgrund ihrer Geometrie eine nicht-lineare Kraft-Weg-Kennlinie aufweisen. Ferner ist es möglich einen Dämpfungsvorsprung 31 derart auszuformen, dass er die Form einer Pyramide, einer Kuppel oder eines Kegels bzw. Kegelstumpfes aufweist.

In dem in Figur 3 gezeigten Halterungselement 30 ist die Auflagefläche der Dämpfungsvorsprünge 31 bei einer Vibration klein im Verhältnis zu einer Auflagefläche der Oberfläche der Kompressionseinrichtung 10 bei einem harten Stoß bzw. Sturz. Dies ist auf die höhere Kraftwirkung zurückzuführen, die bei einem Sturz bzw. einem harten Stoß im Vergleich zu einer Vibration auftritt. Die Auflagefläche nimmt dabei jedoch aufgrund der nicht linearen Kraft-Weg-Kennlinie nicht linear mit der Kraft zu.

## Patentansprüche

1. Inhalationsgerätekompressor mit
- einer mittels Elektromotor (11) angetriebenen Kompressionseinrichtung (10) und
- einem Gehäuse (20) mit
- einer Aufnahmeeinrichtung (21) für eine Kompressionseinrichtung (10) und
- einem Halterungselement (30) zum Haltern der Kompressionseinrichtung (10) in der Aufnahmeeinrichtung (21),
o das an der Oberfläche der Kompressionseinrichtung (10) angreift und
o das eine Mehrzahl von Dämpfungsvorsprüngen (31) aufweist, die jeweils eine derart nichtlineare Kraft-Weg-Kennlinie aufweisen, dass das Kraft/Weg-Verhältnis bei einer Auslenkung der Kompressionseinrichtung (10) zunimmt,
wobei das Halterungselement (30) die Kompressionseinrichtung (10) entlang wenigstens eines Teilumfangs angreifend umschließt.

2. Inhalationsgerätekompressor gemäß Anspruch 1, **dadurch gekennzeichnet, dass** die Dämpfungsvorsprünge (31) Erstreckungsachsen (32) aufweisen, die im wesentlichen senkrecht zur Oberfläche der Kompressionseinrichtung (10) ausgerichtet sind.

3. Inhalationsgerätekompressor gemäß einem der vorangegangenen Ansprüche, **dadurch gekennzeichnet, dass** sich die Dämpfungsvorsprünge (31) in einem Bereich entlang ihrer Erstreckungsachse (32) in Richtung der Oberfläche der Kompressionseinrichtung (10) verjüngen.

4. Inhalationsgerätekompressor gemäß einem der vorangegangenen Ansprüche, **dadurch gekennzeichnet, dass** die am meisten verjüngten Bereiche (33) der Dämpfungsvorsprünge (31) ausgelegt sind, an der Kompressionseinrichtung (10) anzugreifen.

5. Inhalationsgerätekompressör gemäß einem der vorangegangenen Ansprüche, **dadurch gekennzeichnet, dass** die Dämpfungsvorsprünge (31) in Richtung ihrer Erstreckungsachse (32) verformbar ausgebildet sind.

6. Inhalationsgerätekompressor gemäß einem der vorangegangenen Ansprüche, **dadurch gekennzeichnet, dass** die Dämpfungsvorsprünge (31) im wesentlichen aus einem elastischen Material, vorzugsweise einem der Materialen Silikon, EPDM oder TPE bestehen.

7. Inhalationsgerätekompressor gemäß einem der vorangegangenen Ansprüche, **dadurch gekennzeichnet, dass** die Dämpfungsvorsprünge (31) derart nebeneinander angeordnet sind, dass sie im wesentlichen in Form einer Welle in Richtung des Umfangs bzw. Teilumfangs der Kompressionseinrichtung (10) ausgestaltet sind.

8. Inhalationsgerätekompressor gemäß einem der vorangegangenen Ansprüche, **dadurch gekennzeichnet, dass** das Halterungselement (30) die Kompressionseinrichtung (10) entlang eines Umfanges im wesentlichen vollständig angreifend umfasst.

9. Inhalationsgerätekompressor gemäß einem der vorangegangenen Ansprüche, **dadurch gekennzeichnet, dass** das Halterungselement (30) einstückig mit einer im wesentlichen kreisförmigen Öffnung (34) und mit in der Öffnung (34) angeordneten und nach innen gerichteten Dämpfungsvorsprüngen (31) ausgestaltet ist.

## Claims

1. Inhalation apparatus compressor having
- a compression device (10) driven by means of electromotor (11) and
- a housing (20) having
- a receiving device (21) for a compression device (10) and
- a mounting element (30) for mounting the compression device (10) in the receiving device (21),
° which acts on the surface of the compression device (10) and
° which has a plurality of damping projections (31) which have in each case such a non-linear force-path characteristic that the force/path ratio increases during a deflection of the compression device (10),
wherein the mounting element (30) encloses the compression device (10) while acting on at least one part periphery.

2. Inhalation apparatus compressor according to claim 1, **characterised in that** the damping projections (31) have extension axes (32) which are aligned essentially vertically to the surface of the compression device (10).

3. Inhalation apparatus compressor according to one of the preceding claims, **characterised in that** the damping projections (31) taper in a region along their extension axis (32) in the direction of the surface of the compression device (10).

4. Inhalation apparatus compressor according to one of the preceding claims, **characterised in that** the mostly tapered regions (33) of the damping projections (31) are designed to act on the compression device (10).

5. Inhalation apparatus compressor according to one of the preceding claims, **characterised in that** the damping projections (31) are designed to be deformable in the direction of their extension axis (32).

6. Inhalation apparatus compressor according to one of the preceding claims, **characterised in that** the damping projections (31) consist essentially of a resilient material, preferably one of the materials silicone, EPDM or TPE.

7. Inhalation apparatus compressor according to one of the preceding claims, **characterised in that** the damping projections (31) are arranged next to one another such that they are organised essentially in the form of a wave in the direction of the periphery or part periphery of the compression device (10).

8. Inhalation apparatus compressor according to one of the preceding claims, **characterised in that** the mounting element (30) encloses the compression device (10) while acting essentially completely on a periphery.

9. Inhalation apparatus compressor according to one of the preceding claims, **characterised in that** the mounting element (30) is organised to be integral with an essentially circular opening (34) and with damping projections (31) arranged in the opening (34) and directed inwards.

## Revendications

1. Compresseur d'appareil d'inhalation avec :
- un dispositif de compression (10) fonctionnant au moyen d'un moteur électrique (11), et
- un boîtier (20) avec :
- un dispositif formant logement (21) pour un dispositif de compression (10), et
- un élément de fixation (30) pour fixer le dispositif de compression (10) dans le dispositif formant logement (21),
o lequel élément de fixation saisit la surface du dispositif de compression (10) et
o lequel élément de fixation comporte plusieurs parties en saillie faisant amortissement (31) qui présentent chacune une caractéristique effort - déformation non linéaire telle que le rapport effort/déformation augmente lors d'un déplacement du dispositif de compression (10),
l'élément de fixation (30) entourant le dispositif de compression (10) en le saisissant le long d'au moins une partie de son pourtour.

2. Compresseur d'appareil d'inhalation selon la revendication 1, **caractérisé en ce que** les parties en saillie faisant amortissement (31) comportent des axes d'extension (32) qui sont dirigés essientiellement perpendiculairement à la surface du dispositif de compression (10).

3. Compresseur d'appareil d'inhalation selon l'une des revendications précédentes, **caractérisé en ce que**, dans une zone le long de leur axe d'extension (32), les parties en saillie faisant amortissement (31) se rétrécissent en direction de la surface du dispositif de compression (10).

4. Compresseur d'appareil d'inhalation selon l'une des revendications précédentes, **caractérisé en ce que** les zones les plus rétrécies (33) des parties en saillie faisant amortissement (31) sont conçues pour saisir le dispositif de compression (10).

5. Compresseur d'appareil d'inhalation selon l'une des revendications précédentes, **caractérisé en ce que** les parties en saillie faisant amortissement (31) sont conçues de façon pouvoir être déformées dans la direction de leur axe d'extension (32).

6. Compresseur d'appareil d'inhalation selon l'une des revendications précédentes, **caractérisé en ce que** les parties en saillie faisant amortissement (31) sont essientiellement en un matériau élastique, de préférence en l'un des matériaux suivants : silicone, EPDM ou TPE.

7. Compresseur d'appareil d'inhalation selon l'une des revendications précédentes, **caractérisé en ce que** les parties en saillie faisant amortissement (31) sont agencées les unes à côté des autres de telle sorte qu'elles dessinent essientiellement la forme d'une onde en direction du pourtour ou de la partie de pourtour du dispositif de compression (10).

8. Compresseur d'appareil d'inhalation selon l'une des revendications précédentes, **caractérisé en ce que** l'élément de fixation (30) entoure le dispositif de compression (10) en le saisissant presque complètement le long de son pourtour.

9. Compresseur d'appareil d'inhalation selon l'une des revendications précédentes, **caractérisé en ce que** l'élément de fixation (30) est conçu d'un seul tenant avec une ouverture (34) essientiellement circulaire et avec des parties en saillie faisant amortissement (31) agencées dans l'ouverture (34) et dirigées vers l'intérieur.
